# FASCICULE DE BREVET EUROPEEN

(11) **EP 1 378 491 B1**
(45) Date de publication et mention de la délivrance du brevet: **23.08.2006**
(21) Numéro de dépôt: 03358007.7
(22) Date de dépôt: 18.06.2003
(51) Int. Cl.: C02F 1/10, A23L 1/30, B01D 1/18

(54) **Procédé de traitement des eaux de végétation de fruits oléagineux additionnées de biopolymère et poudre d'atomisation obtenue**
Verfahren zur Behandlung der Abwässer ölhaltiger Früchte unter Zusatz eines Biopolymeren und daraus beim Versprühen erhaltenes Pulver
Process of treating wastes of oil containing fruits with biopolymer additive and powder produced by spraying it

(30) Priorité: 01.07.2002 FR 0208181
(43) Date de publication de la demande: 07.01.2004
(73) Titulaire: CENTRE DE COOPERATION INTERNATIONAL EN RECHERCHE AGRONOMIQUE POUR LE DEVELOPPEMENT, 75016 Paris (FR)
(72) Inventeur: Pina, Michel, 34080 Montpellier (FR); Guyot, Bernard, 34090 Montpellier (FR); Graille, Jean, 34070 Montpellier (FR); Figueroa-Espinoza, Maria-Cruz, 34270 Lauret (FR); Barea, Bruno, 34000 Montpellier (FR)
(74) Mandataire: Domange, Maxime

(56) Documents cités:
- EP-A- 1 103 192
- WO-A-00/64282
- DE-A- 3 720 408
- FR-A- 2 624 737

## Description

La présente invention concerne un procédé de traitement des eaux de végétation générées par la transformation des fruits oléagineux contenant des substances organiques d'intérêt consistant en des composés polyphénoliques à effet anti-oxydant. Plus particulièrement, la présente invention concerne le traitement des margines issues des eaux de végétation de l'olive.

La présente invention concerne également un procédé de préparation d'un produit alimentaire intermédiaire contenant des composés polyphénols à effet anti-oxydant ainsi que des produits alimentaires intermédiaires obtenus et des produits alimentaires contenant desdits produits alimentaires intermédiaires.

Les margines constituent un co-produit de l'huilerie d'olive, issu des eaux de végétation des olives additionnées d'une quantité d'eau potable. La teneur en eau des olives arrivées à maturité est de l'ordre de 50%. Si l'on considère que la quantité d'eau additionnée est de l'ordre de 50 kg pour 100 kg d'olives, la quantité de margines est de l'ordre de 70 kg. Le déficit apparent en eau s'explique par la forte rétention d'eau par les grignons. Les grignons sont eux-mêmes constitués des débris solides issus du broyage des olives ; ils sont composés de débris de noyaux et de tissus divers représentant environ 30% de la matière sèche des olives mais se retrouvent hydratés à près de 100%, ce qui conduit à 60 kg de grignons humides environ.

Le tableau 1 ci-après donne la composition moyenne des olives et le tableau 2 ci-après donne la composition moyenne des margines.

**Tableau 1 :**

| CONSTITUTION DES OLIVES | |
|---|---|
| % en poids | |
| Eau | 50 |
| Noyaux | 28 |
| Huile | 22 |

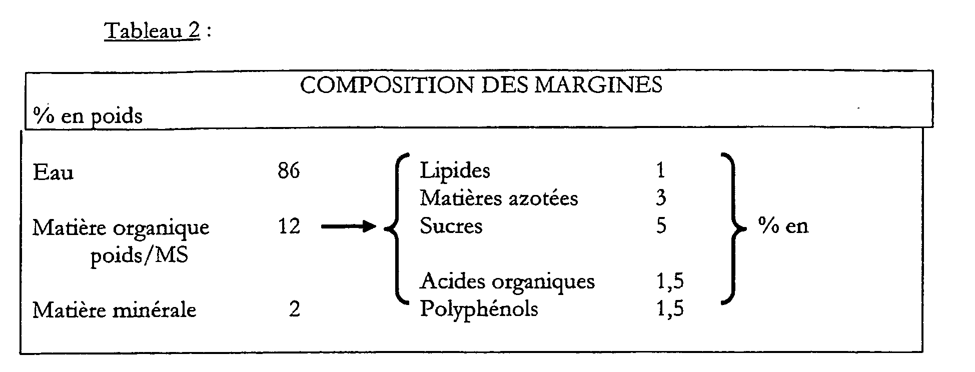

Traditionnellement, les margines étaient épandues dans le sol. Mais ces épandages sont considérés désormais comme une source de pollution importante et ne sont plus autorisés.

C'est pourquoi depuis plus de 50 ans, de nombreuses solutions ont été proposées pour se débarrasser totalement et astucieusement des margines. Malheureusement aucune solution à ce jour n'a donné satisfaction compte tenu du coût qu'elle représente.

On peut citer par exemple l'utilisation des margines comme milieu de culture de micro-organisme pour la fabrication d'enzymes pectinolytiques avec la souche *Crytococcus albidus* (Hamdi M. "Industrial microbiology is useful in the re-use and treatment of olive mill wastewater", Olivae, 1993, 46, 20-25) ou comme milieu de culture pour la production de protéines d'organismes unicellulaires comme celles de *Torulopsis utilis* (Carola C., By-products, In Olive Oil Technology, JM Moreno Martinez, ed. FAO Rome, 1975, 77-87).

Pour la culture des levures il est nécessaire d'ajouter une source d'azote comme l'ammonium sulfate.

On a aussi proposé l'utilisation des margines comme engrais pour les oliveraies mais cela pose semble-t-il des problèmes agronomiques, des risques de pollution et des modifications mécano-physicochimiques des sols (Ranalli A. "The effluent from olive mills: proposals for re-use and purification with reference to Italian legislation. Part I", Olivae, 1991, 37, 30-35 ; Paredes M.J., Moreno A., Cormenzance R. et Martinez J. "Characteristic of soil after pollution with waste waters from olive oil extraction plants" Chemosphere, 1987, 16, 1557-1563).

On peut citer enfin l'introduction des margines dans un foyer de chaudière adaptée pour la production de vapeur ; les matières organiques fournissent un peu d'énergie mais les matières minérales obligent à utiliser des brûleurs spéciaux sinon ces derniers se dégradent en laitiers avec les sels minéraux (Ranalli A. "The effluent from olive mills: proposals for re-usc and purification with reference to Italian legislation. Part II", Olivae, 1991, 38, 26-31).

En conclusion on constate que tous les procédés proposés à ce jour sont des procédés compliqués, économiquement onéreux et qui pour certains revêtent d'autres inconvénients différents de ceux des margines initiales.

D'autre part, on sait que l'olive est un fruit particulièrement intéressant en ce sens qu'elle contient de nombreuses espèces moléculaires du type phénol. Ces composés sont réputés pour leurs activités biologiques potentielles comprenant l'activité antioxydante (Visioli F. et Galli C. "Olive oil phenols and their potential effects on human health" J. Agric. Food Chem., 1998, 46, 4292-4296 et "The effect of minor constituents of olive oil on cardiovascular disease: new findings" Nutr. Rev., 1998, 56, 142-147).

Du fait du coefficient de partage huile/eau largement en faveur de la phase aqueuse et des quantités d'eau importantes apportées au milieu, les phénols initialement présents dans l'olive sont retrouvés majoritairement dans les margines et sont ainsi perdus. Dans le tableau 3, on donne, à titre indicatif, la liste des composés phénoliques principaux avec leur concentration selon les travaux de Visioli F., Romani A., Molinacci N., Zarine S., Conte D., Vincieri F. et Galli C. "Antioxidant and other biological activities of olive mill waste waters.",J. Agric. Food Chem., 1999, 47, 3397-3401.

**Tableau 3 :**

| Composé | g/100g de matière sèche |
|---|---|
| Hydroxytyrosol | 1,56 |
| Tyrosol | 0,85 |
| Acide élénolique | 4,30 |
| Dérives de l'oleuropeine | 0,50 |
| Lutéoline 7-Glucoside | 0,22 |
| Quercétine | 0,13 |
| Dérivés de l'acide cinnamique | 0,55 |
| Polyphénols totaux | 8,11 |

Les olives contiennent plus particulièrement deux molécules phénoliques bioactives, le tyrosol et l'hydroxytyrosol. Ces molécules sont des antioxydants dont on connaît l'intérêt pour la santé humaine. Les antioxydants sont en effet reconnus comme des agents piégeant les entités radicalaires à oxygène actif ; ces radicaux libres sont impliqués dans l'étiologie de diverses maladies chez l'homme (Aruoma O. I. "Extracts as antioxydants prophylatic agents" Int. News Fats, Oil Relat. Matter, 1997, 8, 1236-1242). Ces radicaux libres sont également responsables de la dégradation des denrées alimentaires et des produits cosmétiques.

Un problème à la base de l'invention est donc également de fournir un procédé permettant de récupérer et valoriser ces composés biologiquement actifs que sont les composés phénoliques antioxydants des margines.

Plus particulièrement, un but de la présente invention est de fournir un procédé permettant de récupérer des composés polyphénols très sensibles du fait de leur susceptibilité à l'oxydation, à la volatilité et/ou à toute autre perturbation altérante contenue dans des solutions aqueuses notamment des eaux de végétation générées pendant la transformation des fruits oléagineux.

Un autre but de la présente invention est de fournir un procédé de récupération de composés polyphénoliques biologiquement actifs qui soient simples à réaliser et avantageux économiquement.

En effet, très souvent lorsque l'on veut récupérer des composés polyphénols biologiquement actifs dans ce type d'extraits aqueux, on met en oeuvre des procédés de séparation complexes et coûteux qui engendrent de plus des pertes importantes en composés que l'on voudrait précisément récupérer.

Pour ce faire, la présente invention fournit un procédé de traitement d'un extrait aqueux d'origine végétale contenant des composés polyphénoliques à effet anti-oxydant, caractérisé en ce qu'on réalise les étapes comme définies dans la revendication 1c-à d dans lesquelles :
a) on ajoute dans lesdites eaux de végétation un biopolymère de nature polysaccharidique (PS) d'origine végétale ou animale, et un agent émulsifiant, et
b) on soumet le mélange obtenu à un procédé d'atomisation permettant d'obtenir un atomisât sous forme de poudre contenant la totalité de la matière organique ou minérale comprise dans lesdites eaux de végétation encapsulée au sein d'une matrice protectrice dudit biopolymère, y compris lesdits composés polyphénolidues, lesquels ne sont pas dégradés.

Plus particulièrement, dans le procédé selon l'invention, à l'étape a), le rapport pondéral dudit biopolymère par rapport audit extrait sec mis en oeuvre dans le procédé est compris entre 2 et 8, de préférence entre 3 et 6.

Les inventeurs ont donc découvert qu'il était possible plutôt que de séparer les composés polyphénols biologiquement actifs directement, de mettre en oeuvre un procédé consistant essentiellement à ajouter une charge organique protectrice dans une solution aqueuse en l'espèce, un milieu aqueux d'origine végétale mais qui peut être aussi une émulsion à phase aqueuse continue, puis à atomiser le produit résultant.

Par ce procédé d'atomisation, l'eau est donc évaporée et les composés polyphénols biologiquement actifs, à savoir les substances organiques d'intérêt se trouvent encapsulées dans une matrice protectrice de biopolymères qui préservent ladite substance organique d'intérêt de toute altération, notamment qui a un effet protecteur contre son oxydation.

Les inventeurs ont également découvert que, non seulement ces composés polyphénols sont protégés au sein de la matrice biopolymères par le procédé d'encapsulation selon la présente invention, mais en outre leur teneur est stable au cours du temps.

L'invention est plus particulièrement avantageuse lorsque ladite substance organique d'intérêt est constituée par des composés polyphénoliques à effet antioxydant issus des margines.

A titre de dits biopolymères , on peut citer les biopolymères polysaccharides choisis parmi maltodextrines, les amidons et les chitosanes.

Le procédé d'encapsulation par atomisation (également connu de l'homme de l'Art sous la dénomination "spray drying") est bien connu de l'homme de l'Art et consiste à emprisonner des substances organiques et minérales constitutives d'extraits secs d'une solution aqueuse dans une matrice polymère en suscitant l'évaporation des solvants d'une dispersion dudit polymère en mélange avec un dit extrait aqueux renfermant lesdites substances. Le procédé consiste à pulvériser le mélange aqueux à atomiser en fines gouttelettes de manière former un brouillard véhiculé par de l'air chaud, puis à effectuer l'évaporation de l'eau de manière à transformer les gouttelettes en poudre sèche appelée "atomisat".

Le procédé d'atomisation selon l'étape b) de l'invention est particulièrement avantageux car il est simple à réaliser et peu onéreux. D'autre part, les inventeurs ont découvert que, de façon surprenante, les conditions de températures mises en oeuvre dans ce type de procédé d'atomisation n'induisent pas la destruction des substances polyphénols comme le tyrosol ou l'hydroxyde tyrosol.

En effet, dans un mode de réalisation du procédé d'atomisation selon l'invention, la fourchette de température d'entrée dans l'atomiseur est comprise entre 150 et 250°C et la température de sortie comprise entre 60 et 100°C.

Comme mentionné précédemment, dans un mode de réalisation avantageux, ledit extrait aqueux est constitué par la margine issue des eaux de végétation de l'olive et lesdites substances organiques anti-oxydantes comprennent des composés polyphénols consistant dans le tyrosol et l'hydroxytyrosol.

La présente invention a donc également pour objet, une poudre d'atomisation obtenue par le procédé selon l'invention, caractérisée en ce qu'elle comprend une matrice de dit biopolymère renfermant ladite matière organique ou minérale constitutive dudit extrait sec de l'extrait aqueux et elle comprend une teneur en dite substance polyphénol d'intérêt non dégradé stable au cours du temps.

Dans le cas de traitement de margines, un moulin traitant 800 kg à l'heure, soit 8 tonnes par jour ou encore 720 tonnes en trois mois, représentant la saison, est susceptible de produire 500 tonnes de margines c'est à dire environ 370 tonnes de poudres stabilisées selon l'invention contenant, par exemple, environ 2 à 3% de polyphénols pour le cas des margines traitées et utilisables à tout moment dans des formulations diverses.

Ces poudres selon l'invention peuvent constituer des produits alimentaires intermédiaires du type "alicaments" ou "nutraceutiques" ou des produits cosmétiques compte tenu du fait que les substances polyphénols conservent, le cas échéant, leur propriété organoleptique du fait de l'effet protecteur des biopolymères d'encapsulation.

Les essais réalisés démontrent qu'il est possible, en traitant les margines immédiatement à la sortie de la centrifugeuse, d'encapsuler les polyphénols qu'elles comprennent dans leurs matières sèches et de préserver ainsi ceux-ci de l'oxydation. Compte tenu du débit aléatoire en sortie de la centrifugeuse triphasée, les margines sont de préférence stockées dans une enceinte en acier avec double enveloppe, et le réservoir tampon entre la centrifugeuse et la tour d'atomisation est maintenu réfrigéré vers 10°C soit sous atmosphère d'azote, soit sous forme d'un réservoir à toit flottant, c'est à dire sans espace de tête.

Le procédé selon l'invention permet également d'encapsuler des acides chlorogéniques sans perte, c'est à dire en conservant une concentration stable dans le temps avec des carbohydrates tels que les amidons, les maltodextrines et les chitosanes

Une poudre d'atomisation obtenue par le procédé selon l'invention se caractérise en ce que ladite poudre est très fluide et présente une dispersibilité dans l'eau très homogène. On entend ici par "très fluide" que la poudre se comporte comme un liquide en terme d'écoulement.

Plus particulièrement, une poudre selon l'invention comprend un pourcentage pondéral de dit biopolymère compris entre 20 et 95%, de préférence entre 80 et 90%.

Les produits obtenus selon la présente invention constituent d'excellents produits intermédiaires dans le domaine de l'alimentation, les substances polyphénols d'intérêts pouvant être des arômes ou des nutriments.

La présente invention permet donc d'élaborer des produits alimentaires intermédiaires par atomisation de solutions aqueuses, notamment des eaux de végétation, des extraits, des condensats ou perméats, additionnés d'une charge organique constituée d'un biopolymère, pour la récupération, la stabilisation et la valorisation de substances telles que des antioxydants mais aussi des arômes ou des micro-nutriments. Plus précisement, la présente invention a pour objet un procédé de préparation d'un produit alimentaire contenant des composés polyphénols à effet anti-oxydant, caractérisé en ce que l'on traite un extrait aqueux d'origine végétale par un procédé de traitement d'extraits aqueux selon l'invention et on récupère une poudre d'atomisation selon l'invention à titre dedit produit alimentaire intermédiaire.

La présente invention a donc également pour objet un produit alimentaire intermédiaire contenant des composés polyphénoliques à effet anti-oxydant, caractérisé en ce qu'il comprend une poudre d'aromisation selon l'invention.

La présente invention a également pour objet un procédé de préparation d'un produit alimentaire contenant des composés polyphénols à effet anti-oxydant caractérisé en ce que l'on mélange un produit alimentaire intermédiaire contenant des composés polyphénols à effet anti-oxydant selon la présente invention avec une composition alimentaire.

Dans un mode préféré de réalisation, la teneur pondérale en poudre d'atomisation ne dépasse pas 20%, de préférence 10%, dans ladite composition alimentaire ou cosmétique.

Il est à noter que si de préférencé le procédé de traitement selon l'invention est appliqué sur de la matière fraîche, en sortie de centrifugeuse, notamment en sortie de centrifugeuse de l'huilerie en cas de traitement de margine, ce procédé peut également être appliqué sur un extrait aqueux congelé. Cette possibilité de conservation par congélation de l'extrait aqueux permet de différer le traitement d'atomisation selon la présente invention.

D'autres caractéristiques et avantages de la présente invention apparaîtront à la lumière des exemples qui vont suivre.

### Exemple I

500 ml de margines fraîches contenant 10% de Maltodextrine Glucidex 2B de chez Roquette Frères SA et 2% de caséinate de sodium sont introduits dans un atomiseur Büchi muni d'un nébuliseur à buse à séchage à co-courant (190 minispray drying).
- Temps de traitement : 80 minutes
- Température d'entrée : 160°C
- Température de sortie : 95°C

On obtient une poudre très fluide légèrement colorée, dégageant l'arôme d'olive à la température ambiante (15 à 30°C). Cette propriété se conserve dans le temps et la teneur en polyphénols est constante ; celle du tyrosol et de l'hydroxytyrosol est invariable, à condition de conserver le produit dans des emballages étanches.

Les analyses réalisées par absorption dans l'U.V. démontrent que les polyphénols présents ne s'oxydent pas.

### Exemple II

On réalise une atomisation de margine selon l'exemple I, mais on utilise 10% de Maltodextrine Glucidex 6 de Roquette Frères SA.

On obtient les mêmes résultats que dans l'exemple 1.

### Exemple III

On réalise une atomisation de margine selon l'exemple 1, mais on utilise un atomiseur pilote NIRO avec une solution contenant 60% d'amidon soluble de maïs de chez Roquettes Frères SA.

La température du nébuliseur est de 200°C et celle de la sortie 80°C.

On obtient des résultats identiques à l'exemple I.

### Exemple IV

On réalise une atomisation de margine selon l'exemple III, mais avec 50% de Maltodextrine 2B de chez Roquettes Frères SA.

On obtient des résultats identiques à l'exemple 1.

### Exemple V

Un extrait de café commercial a été atomisé en utilisant une charge composée de maltodextrines avec ou sans ajout d'amidon de riz ou de maïs.

Les résultats montrent que les acides chlorogéniques résistent bien à l'atomisation (T°C d'entrée = 180-200°C, T°C de sortie = 70-80°C) et conservent leurs propriétés antioxydantes (mesurées par le test du 2,2 diphényl-1-picrylhydrazyl (DPPH) (Brand-Williams et al., Lebensm, Wiissen Technol., 1995, 28, 25-30).

### Exemple VI

Fabrication de pain contenant des margines d'huileries d'olive:

Les margines sont récupérées à l'état liquide telles qu'elles sont émises par la centrifugeuse de l'huilerie; Elles sont conservées à -20° C jusqu'au moment de l'emploi Après décongélation pendant 24 heures à +4° C, ces dernières sont réchauffées à la température ambiante (20-25° C) à l'aide d'un bain-marie.

Une poudre atomisée est obtenue à partir d'un mélange de margine décongelée et réchauffée avec des maltodextrines.

Les margines à l'état liquide renferment environ 5 à 10 % de matière sèche. La poudre atomisée séchée, obtenue conformément aux exemples 1 à 4, est constituée en poids d'environ 50% de matière sèche de margines et 50% de maltodextrines. L'humidité de la poudre atomisée est de l'ordre de 3 %. Cette poudre atomisée, obtenue selon les exemples 1 à 4, a été utilisée dans différentes fabrications de pain selon les techniques boulangères mises en oeuvre par l'homme de l'art en boulangerie en ajoutant différentes proportions de 5 à 30% en poids de margine atomisée dans différentes farines.

Les différentes fabrications sont toutes faites sur la base d'un kilo de farine pure ou en mélange avec la poudre atomisée et différents additifs tels que sel, gluten et levure.

La fabrication de pain avec ces farines contenant des atomisats de margine, est possible et ne présente pas de difficulté particulière.

### Résultats :

La fabrication de pains avec l'atomisat de margines est possible à condition de ne pas dépasser, de préférence, 20 % d'atomisat. Toutefois, au-delà de 20% d'atomisats, on peut fabriquer des produits plus denses tels que des gâteaux.

Après dégustation organoleptique, les résultats sont tout à fait satisfaisant. L'amertume n'est plus détectable, ou à peine détectable, et la flaveur d'olive est bien présente bien que légère.

## Revendications

1. Procédé de traitement des eaux de végétation de fruits oléagineux contenant des substances organiques d'intérêt constituées par des composés polyphéooliques à effet anti-oxydant, **caractérisé en ce qu'**on réalise les étapes dans lesquelles :
a) on ajoute dans lesdites eaux de végétation un bio polymère de nature polysaccharidique (PS) d'origine végétale ou animale, et un agent émulsifiant, et
b) on soumet le mélange obtenu à un procédé d'atomisation permettant d'obtenir un atomisât sous forme de poudre contenant la totalité de la matière organique ou minérale comprise dans lesdites eaux de végétation, encapsulée au sein d'une matrice protectrice dudit bio polymère, y compris lesdits composés polyphénoliques, lesquels ne sont pas dégradés.

2. Procédé selon la revendication 1, **caractérisé en ce que** lesdites eaux de végétation comprennent des margines d'olive.

3. Procédé selon la revendication 1 ou 2, **caractérisé en ce que** ledit bio polymère polysaccharide est choisi parmi les maltodextrines, les amidons et les chitosanes.

4. Procédé selon l'une des revendications 1 à 3, **caractérisé en ce que** à l'étape a), le rapport pondéral dudit bio polymère par rapport audit extrait sec mis en oeuvre dans le procédé est compris entre 2 et 8, de préférence entre 3 et 6.

5. Procédé selon l'une des revendications 1 à 4, **caractérisé en ce que**, à l'étape b), la fourchette de température d'entrée dans l'atomiseur est comprise entre 150 et 250°C et la température de sortie comprise entre 60 et 100°C.

6. Poudre d'atomisation obtenue par le procédé selon l'une des revendications 1 à 5, comprenant une matrice de dit bio polymère renfermant ladite matière organique ou minérale constitutive desdites eaux de végétation et elle comprend une teneur en dite substance organique d'intérêt non dégradée stable au cours du temps, **caractérisée en ce que** ladite poudre est très fluide et présente une dispersibilité dans l'eau très homogène.

7. Poudre selon la revendication 6, **caractérisée en ce qu'**elle comprend un pourcentage pondéral de dit bio polymère compris entre 20 et 95 %, de préférence entre 80 et 90 %.

8. Produit alimentaire intermédiaire aromatisé contenant des composés polyphénols à effet anti-oxydant, **caractérisé en ce qu'**il comprend une poudre d'atomisation selon l'une des revendications 6 ou 7.

9. Produit alimentaire selon la revendication 8, **caractérisé en ce qu'**il est constitué d'une farine de pain contenant une dite poudre selon l'une des revendications 6 ou 7.

10. Farine de pain selon la revendication 9, **caractérisée en ce que** ladite poudre est obtenue à partir de margines issues des eaux de végétation de l'olive.

11. Procédé de préparation d'un produit alimentaire contenant des composés polyphénoliques à effet anti-oxydant, **caractérisé en ce que** l'on mélange un produit alimentaire intermédiaire selon la revendication 9 avec une composition alimentaire.

## Claims

1. A method of processing fruit waters of oilfruit containing organic substances of interest constituted by anti-oxidant effect polyphenolic compounds, the method being **characterized by** performing the following steps:
a) adding to said fruit waters a biopolymer of polysaccharide (PS) nature of vegetable or animal origin, and an emulsifying agent; and
b) submitting the resulting mixture to an atomization method enabling an atomized powder to be obtained containing all of the organic or mineral matter contained in said fruit waters encapsulated within a protective matrix of said biopolymer, including said polyphenolic compounds, which are not degraded.

2. A method according to claim 1, **characterized in that** said fruit waters comprise olive black liquors.

3. A method according to claim 1 or claim 2, **characterized in that** said polysaccharide biopolymer is selected from maltodextrins, starches, and chitosans.

4. A method according to any one of claims 1 to 3, **characterized in that** in step a), the ratio by weight of said biopolymer relative to said dry extract produced in the method lies in the range 2 to 8, and preferably in the range 3 to 6.

5. A method according to any one of claims 1 to 4, **characterized in that** in step b), the temperature range at the inlet to the atomizer is 150°C to 250°C, and the outlet temperature lies in the range 60°C to 100°C.

6. Atomization powder obtained by the method according to any one of claims 1 to 5, comprising a matrix of said biopolymer enclosing said organic or mineral material constituting said fruit waters, and having a content of said organic substance of interest that is not degraded and stable over time, **characterized in that** said powder is very fluid and presents very homogenous dispersibility in water.

7. A powder according to claim 6, **characterized in that** it includes a percentage of weight of said biopolymer lying in the range 20% to 95%, and preferably in the range 80% to 90%.

8. A flavored intermediate food product containing anti-oxidant effect polyphenol compounds, the product being **characterized in that** it comprises an atomization powder according to claim 6 or claim 7.

9. A food product according to claim 8, **characterized in that** it is constituted by a bread flour containing a said powder according to claim 6 or claim 7.

10. A bread flour according to claim 9, **characterized in that** said powder is obtained from black liquors coming from olive fruit waters.

11. A method of preparing a food product containing anti-oxidant effect polyphenolic compounds, **characterized in that** an intermediate food product according to claim 9 is mixed with a food composition.

## Patentansprüche

1. Verfahren zur Behandlung der Vegetationswasser ölhaltiger Früchte, die organische Substanzen von Interesse enthalten, bestehend aus polyphenolischen Verbindungen mit Antioxidationswirkung, **dadurch gekennzeichnet, daß** man die Schritte durchführt, in denen:
a) man den Vegetationswassern ein Biopolymer mit Polysaccharidnatur (PS) pflanzlichen oder tierischen Ursprungs und einen Emulgator zugibt und
b) man das erhaltene Gemisch einem Zerstäubungsvorgang unterzieht, der es erlaubt, eine Zerstäubung in Form eines Pulvers zu erhalten, das die Gesamtheit des organischen und mineralischen Materials enthält, das in den Vegetationswassern umfaßt ist, verkapselt in einer Schutzmatrix des Biopolymers, einschließlich der polyphenolischen Verbindungen, die nicht abgebaut sind.

2. Verfahren nach Anspruch 1, **dadurch gekennzeichnet, daß** die Vegetationswasser Olivenpreßreste umfassen.

3. Verfahren nach Anspruch 1 oder 2, **dadurch gekennzeichnet, daß** das Biopolymerpolysaccharid gewählt ist unter den Maltodextrinen, den Stärken und Chitosanen.

4. Verfahren nach einem der Ansprüche 1 bis 3, **dadurch gekennzeichnet, daß** bei Schritt a) das Gewichtsverhältnis des Biopolymers im Verhältnis zum Trockenextrakt, das in dem Verfahren eingesetzt wird, zwischen 2 und 8, vorzugsweise zwischen 3 und 6 liegt.

5. Verfahren nach einem der Ansprüche 1 bis 4, **dadurch gekennzeichnet, daß** bei Schritt b) der Eingangstemperaturbereich im Zerstäuber zwischen 150 und 250 °C und die Ausgangstemperatur zwischen 60 und 100 °C liegt.

6. Zerstäubungspulver, erhalten durch das Verfahren nach einem der Ansprüche 1 bis 5, umfassend eine Matrix des Biopolymers, welche das organische oder mineralische Material einschließt, das die Vegetationswasser bildet und sie einen Gehalt an der organischen Substanz von Interesse umfaßt, die nicht abgebaut und zeitlich stabil ist, **dadurch gekennzeichnet, daß** das Pulver sehr fluid ist und eine Dispergibilität in Wasser aufweist, die sehr homogen ist.

7. Pulver nach Anspruch 6, **dadurch gekennzeichnet, daß** es einen Gewichtsprozentanteil des Biopolymers zwischen 20 und 95%, vorzugsweise zwischen 80 und 90% umfaßt.

8. Aromatisiertes Nahrungsmittelzwischenprodukt, das Polyphenolverbindungen mit Antioxidationswirkung enthält, **dadurch gekennzeichnet, daß** es ein Zerstäubungspulver nach einem der Ansprüche 6 oder 7 umfaßt.

9. Nahrungsmittelprodukt nach Anspruch 8, **dadurch gekennzeichnet, daß** es aus einem Brotmehl besteht, das ein Pulver nach einem der Ansprüche 6 oder 7 enthält.

10. Mehl nach Anspruch 9, **dadurch gekennzeichnet, daß** das Pulver aus Pressresten aus Olivenvegetationswassern erhalten wird.

11. Verfahren zur Herstellung eines Nahrungsmittelproduktes, das Polyphenolverbindungen mit Antioxidationswirkung enthält, **dadurch gekennzeichnet, daß** das Gemisch ein Nahrungsmittelzwischenprodukt nach Anspruch 9 mit einer Nahrungsmittelzusammensetzung ist.
